(19) Europäisches Patentamt
European Patent Office
Office européen des brevets

(11) Publication number : **0 531 175 A2**

(12) # EUROPEAN PATENT APPLICATION

(21) Application number : **92308090.7**

(22) Date of filing : **07.09.92**

(51) Int. Cl.$^5$ : **C08G 65/08,** C08G 75/08, C08G 59/02, C08G 59/32

(30) Priority : **06.09.91 JP 254377/91**

(43) Date of publication of application : **10.03.93 Bulletin 93/10**

(84) Designated Contracting States : **DE FR GB**

(71) Applicant : **NIPPON OIL CO. LTD.
3-12, Nishi Shinbashi 1-chome
Minato-ku Tokyo (JP)**

(72) Inventor : **Kobayashi, Masaaki
88-3, Shinohara-cho, Kohoku-ku
Yokohama-shi, Kanagawa-ken (JP)**
Inventor : **Ikai, Keizo
Towa Gadenhausu Hayama 506, 887-1, Nagae
Hayama-machi, Miura-gun, Kanagawa-ken (JP)**
Inventor : **Matsuno, Mitsuo
12-14, Hino 1-chome, Konan-ku
Yokohama-shi, Kanagawa-ken (JP)**

(74) Representative : **Myerscough, Philip Boyd et al
J.A. Kemp & Co. 14 South Square, Gray's Inn
London WC1R 5LX (GB)**

(54) **Epoxy resins and process for producing same.**

(57) A novel epoxy resin containing a norbornane ring is formed by polymerizing a norbornane compound containing an epoxy group in the ring and an epoxyethyl or epithioethyl group bound to the ring.

This epoxy resin can be cured in a usual manner. The cured product is, because of the norbornane skeleton, excellent in hardness and strength and low in moisture absorption. Compared to an epoxy resin having an aromatic ring, said epoxy resin is excellent in electrical characteristics since it is free from Na, Cl, etc., and is suited for use in sealing materials of LSI, etc. The compound containing the epithioethyl group is high in refractive index because of the epithioethyl group and can be used also in the optical field.

EP 0 531 175 A2

This invention relates to a novel epoxy resin having an epoxy norbornane skeleton and a process for producing same.

PRIOR ART

Epoxy resins having a norbornane skeleton are excellent in hardness and strength and low in moisture absorption. Compared to epoxy resins having an aromatic ring, the above epoxy resins have excellent weatherability and excellent electrical characteristics because they are free from impurities such as Na, Cl, etc., with the result that they are used in sealing materials of LSI and semiconductors, etc. For example, an epoxy resin with a norbornane skeleton having the following structure

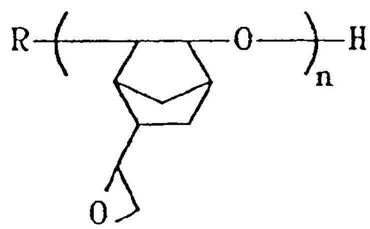

is disclosed in Japanese Laid-open Patent Application (Kokai) No. 9216/1989. The process involved in said document, however, suffers from drawbacks that since a less reactive vinyl group of an oligomer represented by the following formula

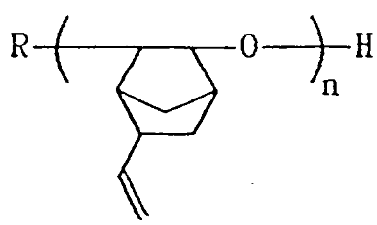

is epoxidized with a peracid, conversion is low and when excess oxidizing agent is used to enhance the conversion, by-products are increased.

Thus, an epoxy resin having an epoxy norbornane skeleton and a process for easily producing same have not been found so far.

MEANS FOR SOLVING THE PROBLEMS

The present inventors have made extensive studies on a process for producing an alicyclic epoxy resin having an epoxy norbornane skeleton, and consequently have found that in a compound containing an epoxyethyl or epithioethyl group and having an epoxy-norbornane skeleton, it is possible to subject the epoxyethyl or epithioethyl group to ring opening polymerization with the epoxy group of the norbornane ring left. This finding has led to completion of this invention.

Namely, this invention relates to an epoxy resim represented by formula (I)

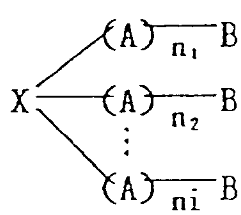

(I)

wherein $n_1$, $n_2$, ..., $n_i$ are each an integer of 0 to 500 and the sum thereof is 1 to 500, B denotes a hydrogen atom, a halogen atom or a hydroxyl group, X denotes a hydrogen atom, a hydroxyl group or a hydrocarbon group, i is a valence of X, and A denotes an alicyclic skeleton having an epoxy group, represented by formula

(II)

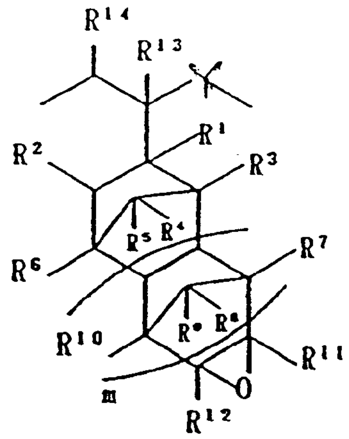

(II)

wherein m is an integer of 0 to 2, Y denotes S or O, and $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ each denote a hydrogen atom or a hydrocarbon group having 1 to 10 carbon atoms.

Moreover, this invention relates to a process for producing an epoxy resin represented by formula (I)

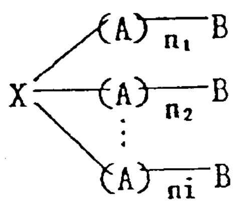

(I)

wherein $n_1$, $n_2$, ..., $n_i$, B, X and A are as defined above,
which comprises polymerizing a compound represented by formula (III)

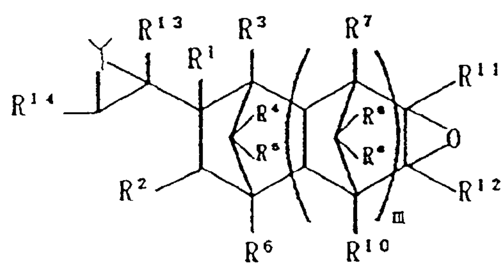

(III)

wherein m, Y, $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ are as defined above.

This invention will be described in more detail below.

In formula (I), $n_1$, $n_2$, ..., $n_i$ are each an integer of 0 to 500, preferably 0 to 300, more preferably 0 to 100, and the sum thereof is 1 to 500, B is a hydrogen atom, a halogen atom or a hydroxyl group, examples of the halogen atom being fluorine, chlorine, bromine and iodine.

X is a hydrogen atom, a hydroxyl group or a hydrocarbon group, and i is a valence of X. Examples of the hydrocarbon group are monovalent and polyvalent hydrocarbon groups. The monovalent hycrocarbon group includes those having usually 1 to 20 carbon atoms, preferably 1 to 12 carbon atoms. Examples thereof are an alkyl group, a cycloalkyl group, an alkenyl group, an aryl group, an aralkyl group, an alkoxy group, an aryloxy group and an aralkyloxy group. Examples of the polyvalent hydrocarbon group are a polyvalent alkane group, a polyvalent cycloalkane group, a polyvalent aryl group, a polyvalent aralkyl group, a polyvalent aryloxy group and a polyvalent aralkyloxy group.

These hydrocarbon groups are at times residues of organometallic compounds used as a precursor in the production (which will be later described). Examples of the organometallic compounds are an alkali metal alkoxide, an alkaline earth metal alkoxide, an alkylated alkali metal, an alkylated alkaline earth metal, an organoaluminum compound represented by the formula

$$RnAlX_{3-n}$$

wherein R denotes a hydrocarbon group such as an alkyl or alkoxy group having 1 to 12 carbon atoms, preferably 1 to 6 carbon atoms, X denotes a halogen atom, and n is $0<n\leqq 3$,

and a Grignard reagent compound represented by the formula

$$R'MgX$$

wherein R′ denotes a hydrocarbon group, for example, an alkyl group having 1 to 12 carbon atoms, such as a methyl group, an ethyl group or the like, or an aryl group such as a phenyl group or the like, and X denotes a halogen atom such as bromine, chlorine, iodine or the like.

To be more concrete, examples of the alkali metal of the alkali metal alkoxide are lithium, sodium, potassium and rubidium. Examples of the alkaline earth metal of the alkaline earth metal alkoxide are bellyrium, magnesium, calcium, strontium and barium. The alkoxy groups of such various metal alkoxides may be derived from monohydric alcohols, polyhydric alcohols or phenols. The monohydric alcohols are those having usually 1 to 20 carbon atoms, preferably 1 to 12 carbon atoms. Concrete examples thereof are methanol, ethanol, propanol, butanol, pentanol, hexanol, octanol and benzyl alcohol. Examples of the polyhydric alcohols are ethylene glycol, diethylene glycol, triethylene glycol, 1,3-butanediol, 1,4-butanediol, pentanediol, 1,6-hexanediol, neopentyl glycol, hydroxypivalic acid neopentyl glycol ester, cyclohexane dimethanol, glycerin, diglycerin, polyglycerin, trimethylolpropane, trimethylolethane, and pentaerythritol. Examples of the phenols are phenol, cresol, catechol, pyrogallol, hydroquinone, hydroquinone monoethyl ether, bisphenol A, bisphenol F, 4,4-dihydroxybenzophenone, bisphenol S, a phenol resin and a cresol novolak resin.

Examples of the alkali metal of the alkylated alkali metal are lithium, sodium, potassium, and rubidium. Examples of the alkaline earth metal of the alkylated alkaline earth metal are beryllium, magnesium, calcium, strontium, and barium. The alkyl group of the alkylated metal compound is an alkyl group having usually 1 to 12 carbon atoms, preferably 1 to 8 carbon atoms. Examples of the alkyl group are a methyl group, an ethyl group, a propyl group, a butyl group, a pentyl group, a hexyl group, a phenyl group, and a benzyl group.

Examples of the organoaluminum compound represented by the above formula are trimethylaluminum, triethylaluminum, tri-n-propylaluminum, tri-i-propylaluminum, tri-i-butylaluminum, diethylaluminum chloride, ethylaluminum dichloride, diethylaluminum chloride, and ethylaluminum sesquichloride.

Examples of the Grignard compound represented by the formula

$$R'MgX$$

are methylmagnesium bromide, ethylmagnesium bromide, propylmagnesium bromide, butylmagnesium bromide, phenylmagnesium bromide, methylmagnesium chloride, ethylmagnesium chloride, propylmagnesium chloride, phenylmagnesium chloride, butylmagnesium chloride, methylmagnesium iodide, ethylmagnesium iodide, propylmagnesium iodide, phenylmagnesium iodide, and butylmagnesium iodide.

A in formula (I) of this invention is an oxyethylene or thioethylene unit having as a pendant group an alicyclic skeleton with an epoxy group, represented by formula (II). In formula (II), m is an integer of 0 to 2, preferably 0 to 1. $R^1$, $R^2$, $R^3$, $R^4$, $R^5$, $R^6$, $R^7$, $R^8$, $R^9$, $R^{10}$, $R^{11}$, $R^{12}$, $R^{13}$ and $R_{14}$ are each a hydrogen atom or a hydrocarbon group having 1 to 10 carbon atoms, preferably 1 to 6 carbon atoms, and they may be the same or different. Examples of the hydrocarbon group are alkyl groups such as a methyl group, an ethyl group, a propyl group and a butyl group, and aryl groups such as a phenyl group and a tolyl group.

The novel epoxy resin represented by formula (I) in this invention can be prepared by polymerizing the epoxy compound having the epithioethyl or epoxyethyl group, represented by formula (III).

In formula (III), m is an integer of 0 to 2, preferably 0 to 1. $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ are each a hydrogen atom or a hydrocarbon group having 1 to 10 carbon atoms, preferably 1 to 6 carbon atoms, and they may be the same or different. Examples of the hydrocarbon group are alkyl groups such as a methyl group, an ethyl group, a propyl group and butyl group, and aryl groups such as a phenyl group and a tolyl group. Concrete examples are the following compounds

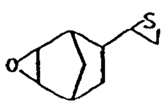 , 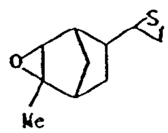 , 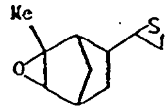 , 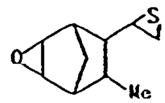 ,

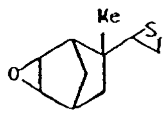 , 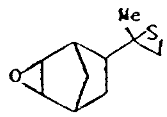 , 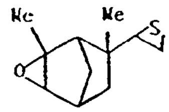 , 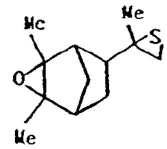 ,

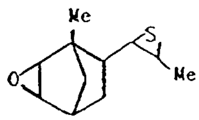 , 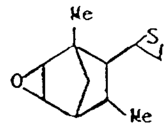 , 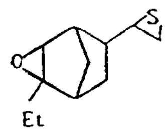 , 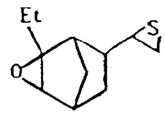 ,

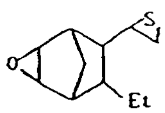 , 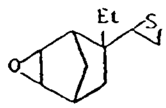 , 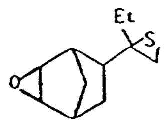 , 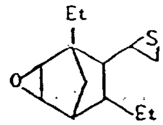 ,

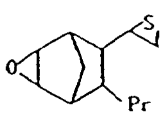 , 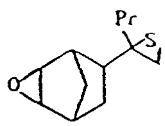 , 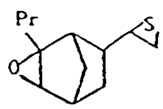 , 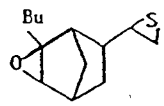 ,

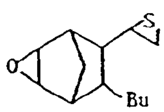 , 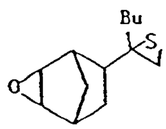 , 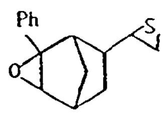 , 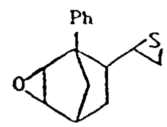 ,

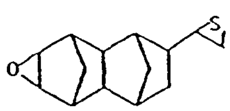 , 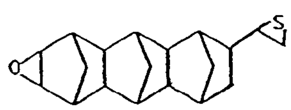

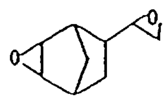, 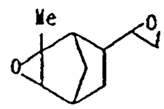, 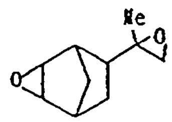, 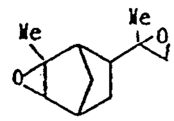,

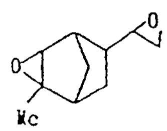, 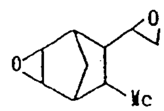, 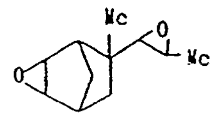, 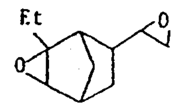,

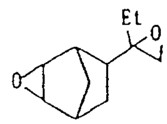, 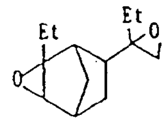, 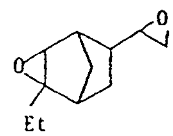, 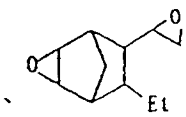,

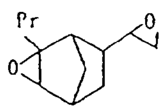, 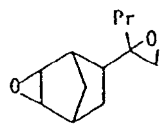, 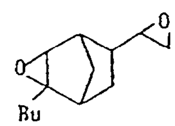, 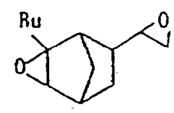,

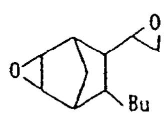, 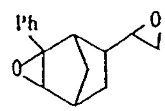, 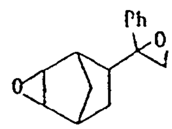, 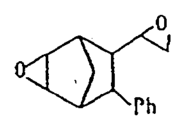,

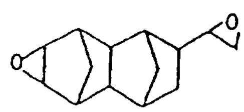, 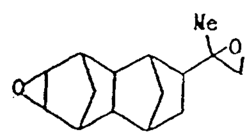,

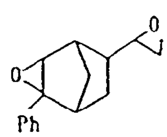

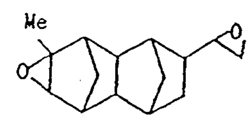, 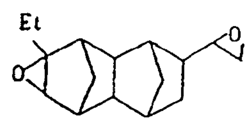,

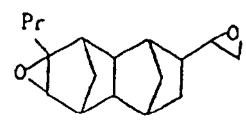, 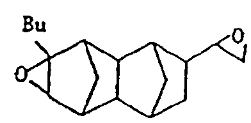,

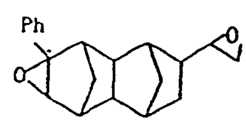, 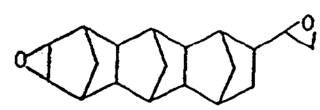

wherein Me=$CH_3$, Et=$C_2H_5$, Pr=$C_3H_7$, Bu=$C_4H_9$, and Ph=$C_6H_5$, and their corresponding epoxyethyl compounds. These epoxy compounds can readily be prepared by a method described in e.g. Japanese Patent Application No. 101572/1990.

Polymerization of a compound (III) with Y=S

When Y is S, the polymerization of the compound represented by formula (III) can usually be carried out in the presence of a catalyst. As the polymerization catalyst used, metals, organometallic compounds, halogenated metals, organic salts and compounds that act as acids are available. As the metals, alkali metals such as lithium, sodium, potassium and rubidium, and alkaline earth metals such as beryllium, magnesium, calcium, strontium and varium are desirous.

Examples of the organometallic compounds are organometallic compounds such as an alkali metal alkoxide, an alkaline earth metal alkoxide, an alkylated alkali metal, an alkylated alkaline earth metal, an organoaluminum compound represented by formula

$$RnAlX_{3-n}$$

wherein R denotes a hydrocarbon group such as an alkyl or alkoxy group having 1 to 12 carbon atoms, preferably 1 to 6 carbon atoms, X denotes a halogen atom, and n is $0<n\leqq 3$,
and a Grignard reagent compound represented by the formula

$$R'MgX$$

wherein R′ denotes a hydrocarbon group, e.g., an alkyl group having 1 to 12 carbon atoms, such as a methyl group, an ethyl group or the like, or an aryl group such as a phenyl group or the like, X denotes a halogen atom such as bromine, chlorine, iodine or the like.

To be more concrete, examples of the alkali metal of the alkali metal alkoxide are lithium, sodium, potassium and rubidium. Examples of the alkaline earth metal of the alkaline earth metal alkoxide are beryllium, magnesium, calcium, strontium and varium. Alkoxy groups of such metal alkoxides may be derived from monohydric alcohols, polyhydric alcohols and phenols. The monohydric alcohols are those having usually 1 to 20 carbon atoms, preferably 1 to 12 carbon atoms. Examples of the monohydric alcohyols are methanol, ethanol, propanol, butanol, pentanol, hexanol, octanol and benzyl alcohol. Examples of the monohydric alcohols are ethylene glycol, diethylene glycol, triethylene glycol, 1,3-butanediol, 1,4-butanediol, pentanediol, 1,6-hexanediol, neopentyl glycol, hydroxypivalic acid neopentyl glycol ester, cyclohexane dimethanol, glycerin, diglycerin, polyglycerin, trimethylolpropane, trimethylolethane, and pentaerythritol. Examples of the phenols are phenol, cresol, catechol, pyrogallol, hydroquinone, hydroquinone monoethyl ether, bisphenol A, bisphenol F, 4,4-dihydroxybenzophenone, bisphenol S, a phenol resin, and a cresol novolak resin.

Examples of the alkali metal of the alkylated alkali metal are lithium, sodium, potassium, and rubidium. Examples of the alkaline earth metal of the alkylated alkaline earth metal are beryllium, magnesium, calcium, strontium, and varium. The alkyl group of the alkylated metal compound is an alkyl group having usually 1 to 12 carbon atoms, preferably 1 to 8 carbon atoms. Examples of the alkyl group are a methyl gruop, an ethyl group, a propyl group, a butyl group, a pentyl group, a hexyl group, a phenyl group, and a benzyl group.

Examples of the organoaluminum compound represented by the formula are trimethylaluminum, triethylaluminum, tri-n-propylaluminum, tri-i-propylaluminum, tri-i-butylalminum, diethylaluminum chloride, ethylaluminum dichloride, diethylaluminum chloride and ethylaluminum sesquichloride.

Examples of the Grignard compound represented by the formula

$$R'MgX$$

are methylmagnesium bromide, ethylmagnesium bromide, propylmagnesium bromide, butylmagnesium bromide, phenylmagnesium bromide, methylmagnesium chloride, ethylmagnesium chloride, propylmagnesium chloride, phenylmagnesium chloride, butylmagnesium chloride, methylmagnesium iodide, ethylmagnesium iodide, propylmagnesium iodide, phenylmagnesium iodide, and butylmagnesium iodide.

When the alkali metal alkoxide, the alkaline earth metal alkoxide, the alkylated alkali metal or the alkylated alkaline earth metal is used as the catalyst, the organometallic compound alone can be used sufficiently, but a compound coordinated with the metal may be added to increase the catalytic activity.

Examples of the compound coordinated with the metal are crown ethers such as 12-crown-4, 14-crown-4, 15-crown-5, 18-crown-6, 21-crown-7, 24-crown-8, 27-crown -9, 30-crown-10, dibenzo-12-crown-4, dibenzo-14-crown -4, dibenzo-15-crown-5, dibenzo-18-crown-6, dibenzo-21-crown-7, dibenzo-24-crown-7, dibenzo-27-crown -9, dibenzo-30-crown-10, benzo-12-crown-4, benzo-15 -crown-5, benzo-18-crown-6, tribenzo-18-crown-6, tetrabenzo-24-crown-8, dicyclohexyl-12-crown-4, dicyclohexyl-14-crown-4, dicyclohexyl-15-crown-5, dicyclohexyl-18-crown-6, dicyclohexyl-21-crown-7, dicyclohexyl-24-crown-8, dicyclohexyl-27-crown-9, dicyclohexyl-30-crown-10, tricyclohexyl-18-crown-6, and tetracyclohexyl-24-crown-8; and amines such as tetramethylethylenediamine and porphylin. The amount of the compound coordinated with the metal is usually about 1 to 100 mols, preferably about 1 to 10 mols per mol of the metal.

Examples of the halogenated metal are trifluoroboron, trichloroboron, trichloroaluminum, tribromoaluminum, pentachloroantimony, trichloroiron, dichloroiron, tetrachlorotitanium, tetrachlorotin, tetrabromotin, pentachloromolybdenum, hexachlorotangsten, and pentafluorophosphorus. They may be used as complexes with

ethers, alcohols, phenol or acetic acid. It is advisable that an active hydrogen-containing compound is co-existent to prevent gelation of the product. As the active hydrogen-containing compound, alcohols, phenols, carboxylic acids, amines and thiols are available. Examples of the alcohols are monohydric alcohols such as methanol, ethanol, propanol, butanol, pentanol, hexanol, octanol, and benzyl alcohol; and polyhydric alcohols such as ethylene glycol, diethylene glycol, triethylene glycol, polyethylene glycol, propylene glycol, dipropylene glycol, 1,3-butanediol, 1,4-butanediol, pentanediol, 1,6-hexanediol, neopentyl glycol, hydroxypivalic acid neopentyl glycol ester, cyclohexanedimethanol, glycerin, diglycerin, polyglycerin, trimethylolpropane, trimethylolethane, and pentaerythritol.

Examples of the phenols are phenol, cresol, catechol, pyrogallol, hydroquinone, hydroquinone monomethyl ether, bisphenol A, bisphenol F, 4,4′-dihydroxybenzophenone, bisphenol S, a phenol resin, and a cresol novolak resin.

Examples of the carboxylic acids are formic acid, acetic acid, propionic acid, butyric acid, fatty acids of animals and plants, fumaric acid, maleic acid, adipic acid, dodecandioic acid, trimellytic acid, pyromellitic acid, polyacrylic acid, phthalic acid, isophthalic acid, and terephthalic acid.

Examples of the amines are monomethylamine, dimethylamine, monoethylamine, diethylamine, propylamine, monobutylamine, dibutylamine, pentylamine, hexylamine, cyclohexylamine, octylamine, dodecylamine, 4,4′-diaminodiphenylmethane, isophoronediamine, xylenediamine, diethylenetriamine, triethylenetetramine, ethanolamine, ethylenediamine, and tetramethylethylenediamine.

Examples of the thiols are mercaptans such as methyl mercaptan, ethyl mercaptan, propyl mercaptan, and phenyl mercaptan, mercaptopropionic acid, and polyhydric alcohol esters such as ethylene glycol dimercaptopropionic acid ester, trimethylolpropane trimercaptopropionic acid, and pentaerythritol pentamercaptopropionic acid.

Examples of the active-hydrogen containing compound are polyvinyl alcohol, partially hydrolyzed polyvinyl acetate, starches, celluloses, cellulose acetate, cellulose acetate butyrate, hydroxyethyl cellulose, acrylic polyol starches, a styrene-allyl alcohol copolymer resin, a styrene-maleic acid copolymer resin, an alkyd resin, a polyester polyol resin, a polyester carboxylic acid resin, polypropylene polyol, and polytetramethylene glycol.

The amount of the active hydrogen-containing compound is usually 0.5 to 100 times, preferably 1 to 20 times that of the halogenated metal.

The organic salts are represented by formula (IV).

$$R^+.Q^- \qquad (IV)$$

wherein $R^+$ is $Ph_3C^+$ (triphenylmethyl ion), $C_7H_7^+$ (tropylium ion) or $CH_3CO^+$ (acetyl ion), and Q is $ClO_4^-$, $SbCl_6^-$, $SnCl_5^-$, $AlCl_4^-$ or $BF_4^-$.

Examples of the compounds that act as acids are hydrochloric acid, sulfuric acid, and p-toluenesulfonic acid; water content, and oxygen are also available.

The amount of the catalyst is usually 0.01 to 50 mol%, preferably 0.1 to 40 mol%.

It is advisable that the polymerization reaction of the compound represented by formula (III) is carried out at a temperature of -70 to 200°C, preferably -20 to 50°C. When the temperature is lower than the above temperature range, the reaction slowly proceeds or hardly proceeds; it is thus not practical. When the temperature is higher than the above temperature range, the reaction is hardly controlled or the heating step has to be contrived; it is thus not practical either. Although the reaction time is not particularly limited, it is usuaslly 0.5 to 500 hours, preferably 1 to 120 hours.

The reaction is usually conducted in a solvent. Although the reaction is not particularly limited, alcohols, aliphatic hydrocarbons, aromatic hydrocarbons, ethers and halogen compounds which are not reacted with the catalyst are available.

Examples of the aliphatic hydrocarbons are pentane, n-hexane, heptane, and octane. Examples of the aromatic hydrocarbons are benzene, toluene, and xylene. Examples of the ethers are diethyl ether, diisopropyl ether, THF, and dioxane. Examples of the halogen compounds are methylene chloride, chloroform, carbon tetrachloride, dichloroethane, trichloroethane, and tetrachloroethane. The other compounds are acetone, ethyl acetate, dimethylformamide, acetonitrile, and dimethyl sulfoxide.

The molecular weight of the product (I) can be adjusted by optionally combining the catalyst, the solvent and the reaction temperature. The molecular weight of the compound of formula (I) is usually 300 to 100,000, preferably 1,000 to 20,000.

<u>Polymerization of a compound (III) with Y=O</u>

When Y is O in formula (III), the polymerization of the compound (III) is carried out in the presence of an anionic polymerization catalyst.

As the anionic polymerization catalyst, an organometallic compound, i.e., the organometallic compound

which is a precursor of the organometallic compound residue of formula (I) is used. Examples of the organometallic compound are as shown above. When using, of these, the alkali metal alkoxide, the alkaline earth metal alkoxide, the alkylated alkali metal or the alkylated alkaline earth metal as the catalyst, the organometallic compound alone can be used sufficiently, but a compound coordinated with the metal may be added to increase the catalytic activity, as is done in polymerizing the compound of formula (III) with Y=S, i.e., the compound of formula (III) having the epithioethyl group.

The other conditions such as a polymerization temperature, a polymerization time, a solvent, etc. may be the same as in polymerizing the compound of formula (III) with Y=S.

## EFFECTS OF THE INVENTION

The epoxy resin represented by formula (I) in this invention features that because of the norbornane skeleton, it is excellent in hardness and strength and low in moisture absorption, as well as excellent in weatherability compared to the epoxy resin having the aromatic ring and in electrical characteristics since it does not contain impurities such as Na, Cl, etc. Further, this resin can be used in sealing materials of LSI, semiconductors, etc. by being crosslinked with a curing agent. Still further, when Y is S, sulfur is high in atomic refractive index; where sulfur is present in a molecule, a refractive index of the molecule increases. Thus, the epoxy resin with Y=S can be applied to an optical usage. Furthermore, the epoxy resin of this invention can easily be prepared by polymerizing the compound of formula (III). In addition, the epoxy resin of this invention has merits that it can be prepared under moderate conditions without heat generation.

## EXAMPLES

The following Synthesis Examples and Examples illustrate this invention in more detail below, but this invention is not limited thereto at all.

## SYNTHESIS EXAMPLE 1

As an example of compound (II), 6-epithioethyl-3-oxatricyclo[3.2.1.0$^{2,4}$]octane can be formed from 6-epoxyethyl-3-oxatricyclo[3.2.1.0$^{2,4}$]octane according to Japanese Patent Application No. 92114/1990 as described below.

Namely, a 200 ml flask was charged with 6-epoxyethyl-3-oxatricyclo[3.2.1.0$^{2,4}$]octane (42 g, 0.28 mol), ammonium thiocyanate (23 g, 0.30 mol) and tetrahydrofuran (125 ml), and they were stirred with a magnetic stirrer in a hot water bath of 50°C. At first, the reaction solution was completely uniform, but about 30 minutes later, the white precipitate was formed. Five hours later, the reaction mixture was suction-filtered with a Nutsche funnel to remove the white precipitate, and the filtrate was concentrated with a rotary evaporator. Toluene (250 ml) and water (80 ml) were added to the concentrate to separate an aqueous layer, followed by washing a toluene layer with water (80 ml x 2). After the toluene layer was dried over magnesium sulfate, toluene was evaporated with a rotary evaporator. The concentrate was distilled in vacuo to obtain 6-epithioethyl-3-oxatricyclo[3.2.1.0$^{2,4}$]octane (40 g, yield 87 %).

## EXAMPLE 1

A nitrogen-purged 100 ml flask was charged with 6-epithioethyl-3-oxatricyclo[3.2.1.0$^{2,4}$]octane (4.0 g, 24 mmols), THF (25 ml), and 18-crown-6 (60 mg, 23 mmols), and they were stirred with a magnetic stirrer. To the mixture was added 0.090 mol/1 of a potassium butoxide-THF solution (1 ml, 0.090 mmol as KOBu), and stirring continued at 15°C. One hundred hours later, water (250 ml) was added to the reaction solution, and the resulting solution was extracted thrice with methylene chloride (60 ml), and the methylene chloride layer was dried over magnesium sulfate. The methylene chloride solution was concentrated to 30 ml, and added dropwise to 300 ml of propyl ether to obtain 3.2 g of a white solid powder. GPC analysis of the white solid powder revealed that it was a polymer having an average molecular weight of 12,000. An IR chart and a $^{13}$CNMR chart of the obtained polymer were shown in Figures 1 and 2.

In the IR chart, absorption of the thioether linkage was observed in 1085 cm$^{-1}$, absorption of the epithio group disappeared in 870 cm$^{-1}$, and intense absorption of the epoxy group in the norbornane ring remained in 849 cm$^{-1}$ and disappeared by hydrolysis with HCl. Accordingly, the product is of the following structure. (n=20)

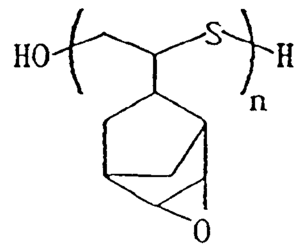

The polymer had a refractive index of 1.586 and an Abbe's number of 54.3.

IR 3494(w), 2964(s), 2876(m), 1462(m), 1446(m), 1379(m), 849(s), 557(m) $cm^{-1}$; $^{13}CNMR(CDCl_3)$ δ 23.5, 23.8, 24.0, 27.4, 33.0, 34.1, 36.2, 36.5, 36.9, 37.2, 37.3, 37.4, 37.6, 39.1, 40.0, 41.1, 42.8, 44.8, 48.7, 49.1, 50.8, 51.0, 51.4, 51.6, 56.1, 77.1, 85.5

Elemental analysis (as $C_9H_{12}OS$ without considering the organic residue of polymer terminals)

| | C(%) | H(%) | O(%) | S(%) |
|---|---|---|---|---|
| found : | 64.7 | 7.3 | 9.2 | 18.8 |
| calculated: | 64.3 | 7.2 | 9.5 | 19.1 |

EXAMPLE 2

A nitrogen-purged 100 ml eggplant type flask was charged with 6-epithioethyl-3-oxatricyclo- [3.2.1.0$^{2.4}$]octane (4.0 g, 24 mmols) and toluene (50 ml), and they were stirred with a magnetic stirrer. To the mixture was added a 1.0 M triethylaluminum-toluene solution (1.0 ml; 1.0 mmol as triethylaluminum), and stirring continued at 20°C. As a result, the reaction solution was found milky. Thirty hours later, water was added to deactivate the catalyst, the white solid was removed from the organic layer by filtration, and the solvent was evaporated from the filtrate. Then, there was obtained 3.8 g of a colorless transparent oil. GPC analysis of the oil revealed that it was a mixture of the polymerized product and 32 % of the starting monomer. When the mixture was dissolved in 20 ml of methylene chloride and the solution was added dropwise to 300 ml of diethyl ether, 2.1 g of a white solid was obtained.

In the IR chart, absorption of the epithio group disappeared in 870 $m^{-1}$, absorption of the thioether linkage was observed in 1080 $cm^{-1}$, and intense absorption of the epoxy group in the norbornane ring remained in 849 $cm^{-1}$ and disappeared by hydrolysis with HCl.

Accordingly, the product is of the following structure. (n=30)

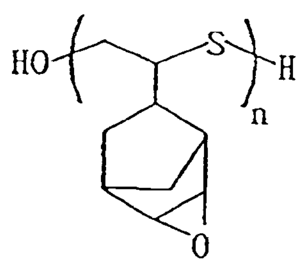

EXAMPLE 3

6-Epithioethyl-3-oxatricyclo[3.2.1.0$^{2,4}$]octane (4.0 g, 24 mmols) and ethanol (5.0 ml, 58 mmols) were charged in a 100 ml flask, and stirred with a magnetic stirrer. To the mixture was added 0.2 mol/l of a $BF_3.OEt_2$-ethyl acetate solution (0.1 ml; 0.020 mmol as $BF_3$) under ice cooling. Two hours later, ethanol and ethyl acetate were evaporated, and methylene chloride and water were added. The methylene chloride layer was separated, dried over magnesium sulfate, concentrated to 5 ml and added dropwise to hexane to obtain 3.1 g of a polymer as a white powder.

EXAMPLE 4

6-Epithioethyl-3-oxatricyclo[3.2.1$^{2,4}$]octane (10.0 g, 59.4 mmols) was heated to 50°C in the presence of air. Two weeks later, there was obtained a solid which was yellow and transparent throughout. GPC analysis of the solid revealed that it was a mixture of the polymerized product and 19 % of the starting monomer. When the mixture was dissolved in ethyl acetate (15 ml) and the solution was added dropwise to hexane, 7.1 g of a polymer as a white powder was formed.

SYNTHESIS EXAMPLE 2

As an example of compound (II), 6-epoxyethyl-3-oxatricyclo[3.2.1.0$^{2,4}$]octane can be formed as follows from 5-vinylbicyclo[2.2.1]hept-2-ene according to Japanese Patent Application No. 101572/1990.

Namely, a three-liter three-necked round-bottomed flask fitted with a stirrer, a pH electrode and a thermometer was charged with 5-vinylbicyclo[2.2.1]-hept-2-ene (43 g, 0.36 mol), methylene chloride (400 ml), acetone (200 ml), a 0.05 M phosphate buffer solution (pH 7.4; 280 ml) and tetrabutylammonium hydrogensulfate (19g, 56 mmols). While cooling the mixture to 10°C, an aqueous solution (water; 2200 ml) of Oxone (a trademark for $2KHSO_5.KHSO_4.K_2HSO_3$ made by E. I. du Pont de Nemours & Co.; 555 g, 1.81 mols as $KHSO_5$) was added thereto dropwise (10 ml/mm). During the reaction, a 5N KOH aqueous solution was added dropwise with a pH controller to adjust pH to 7.2 to 7.8. After the dropwise addition of the Oxone aqueous solution was over, stirring further continued for 4 hours. The reaction solution was suction-filtered with a Nutsche funnel, and $K_2SO_4$ precipitated was separated by filtration. Then, the methylene chloride layer was separated. Said methylene chloride layer was dried over magnesium sulfate and most of methylene chloride was distilled off. The concentrate was passed through a silica gel column to remove tetrabutylammonium hydrogensulfate, followed by conducting simple distillation under reduced pressure. There resulted 6-epoxyethyl-3-oxatricyclo[3.2.1.0$^{2,4}$]octane (46 g, yield 85 %).

EXAMPLE 5

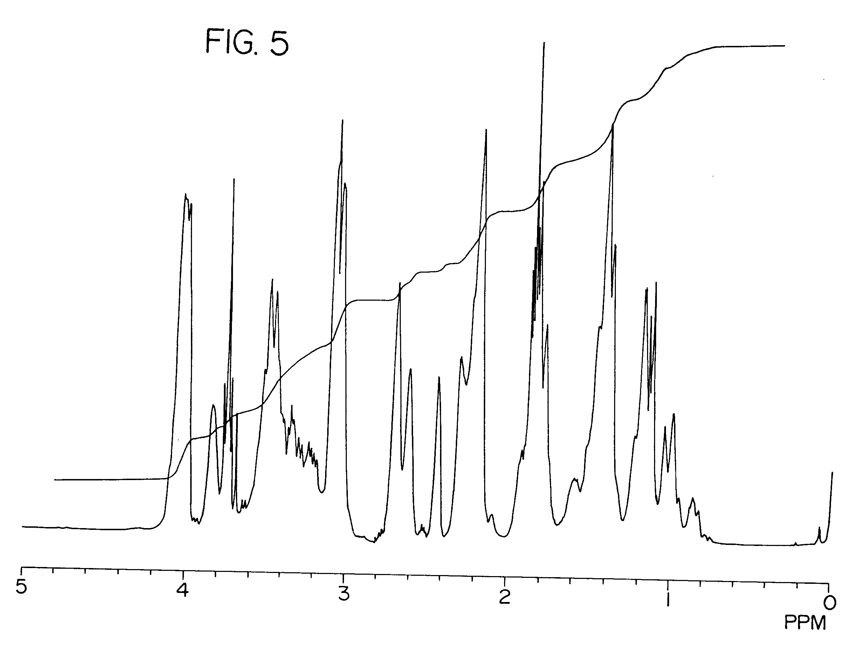

A nitrogen-purged 100 ml flask was charged with 6-epoxyethyl-3-oxatricyclo[3.2.1.0$^{2,4}$]octane (4.5 g, 30 mmols), THF (15 ml) and 18-crown-6 (0.30 g, 1.1 mmols), and they were stirred with a magnetic stirrer. To the mixture was added 0.090 mol/l of a potassium butoxy-THF solution (15 ml, 1.35 mmols as KOBu), and stirring continued at 50°C. One hundred hours later, 5 ml of water was added to the reaction solution, and the resulting solution was stirred. Then, THF was evaporated under reduced pressure, and methylene chloride (50 ml) was added, followed by washing the resulting mixture twice with 50 ml of water. The methylene chloride layer was dried over magnesium sulfate and concentrated to 30 ml. The concentrate was added dropwise to 300 ml of diethyl ether to obtain 3.6 g of a solid as a white powder. An IR chart, a $^{13}$CNMR chart and a $^{1}$HNMR chart of the resulting polymer are shown in Figures 3, 4 and 5.

In the IR chart, absorption of the epoxy group disappeared in 874 cm$^{-1}$, and absorption of the ether linkage was observed in 1065 cm$^{-1}$. Intense absorption of the epoxy group in a norbornane ring remained in 849 cm$^{-1}$ and disappeared by hydrolysis with HCl. Accordingly, the product is of the following structure. (n=30)

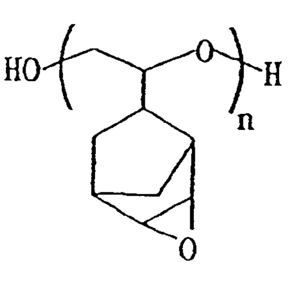

IR 3468(w), 2966(s), 1464(m), 1352(m), 1274(m), 1065(s), 963(m), 924(m), 893(m), 849(s), 735(s), 779(m), 735(s), 702(m) cm$^{-1}$;
$^{13}$CNMR δ 23.7, 24.8, 25.3, 32.6, 33.4, 33.9, 36.7, 37.1, 37.8, 38.0, 38.3, 38.5, 39.1, 39.2, 39.3, 40.1, 42.8, 43.0, 45.5, 51.2, 51.3, 51.7, 67.6, 69.2, 70.7, 80.6, 84.5, 84.6, 84.8, 84.9, 88.3, 88.6, 88.8; $^{1}$HNMR δ 0.99, 1.12, 1.14, 1.17, 1.38, 1.41, 1.78, 1.83, 1.84, 1.85, 1.86, 1.88, 2.19, 2.30, 2.43, 2.61, 2.69, 3.05, 3.08, 3.34, 3.45, 3.48, 3.68, 3.72, 3.77, 3.84, 4.04.

Elemental analysis

| | C(%) | H(%) | O(%) |
|---|---|---|---|
| found : | 70.5 | 8.1 | 21.4 |
| calculated: | 71.0 | 8.0 | 21.0 |

EXAMPLE 6

A nitrogen-purged 100 ml flask was charged with 6-epoxyethyl-3-oxatricyclo[3.2.1.$0^{2,4}$]octane (4.0 g, 24 mmols) and toluene (50 ml), and they were stirred with a magnetic stirrer. To the mixture was added a 1.0 M triethylaluminum-toluene solution (1.0 ml; 1.0 mmol as triethylaluminum), and stirring continued at 20°C. As a result, the reaction solution was found milky. Thirty hours later, water was added to deactivate the catalyst. A white solid was removed from the organic layer by filtration, and the solvent was evaporated from the filtrate. There resulted 3.8 g of a colorless transparent oil. GPC analysis of the oil revealed that it was a mixture of the polymerized product and 32 % of the starting monomer. When the mixture was dissolved in 20 ml of methylene chloride and the solution was added dropwise to 300 ml of diethyl ether, 2.1 g of a white solid was obtained.

In the IR chart, absorption of the epoxy group disappeared in 874 $cm^{-1}$, and absorption of the ether linkage was observed in 1065 $cm^{-1}$. Intense absorption of the epoxy group in the norbornane ring remained in 849 $cm^{-1}$ and disappeared by hydrolysis with HCl.

Accordingly, the product is of the following structure. (n=20)

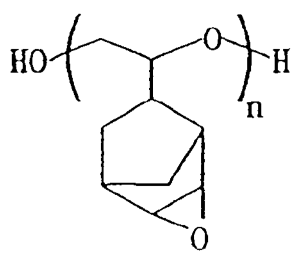

## Claims

1. An epoxy resin represented by formula (I)

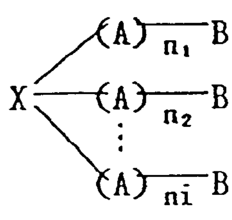

(I)

wherein $n_1$, $n_2$, ..., $n_i$ are each an integer of 0 to 500 and the sum thereof is 1 to 500, B denotes a hydrogen atom, a halogen atom or a hydroxyl group, X denotes a hydrogen atom, a hydroxyl group or a hydrocarbon group, i is a valence of X, and A denotes an alicyclic skeleton having an epoxy group, represented by formula (II)

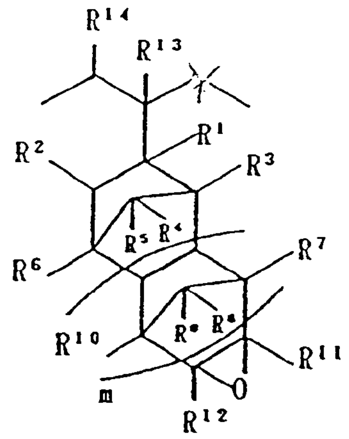

(II)

wherein m is an integer of 0 to 2, Y denotes S or O, and $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ each denote a hydrogen atom or a hydrocarbon group having 1 to 10 carbon atoms.

**2.** A process for producing an epoxy resin represented by formula (I)

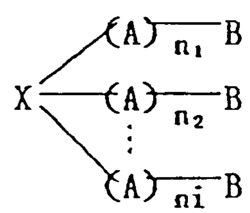

(I)

wherein $n_1$, $n_2$, ..., $n_i$ are each an integer of 0 to 500 and the sum thereof is 1 to 500, B denotes a hydrogen atom, a halogen atom or a hydroxyl group, X denotes a hydrogen atom, a hydroxyl group or a hydrocarbon group, i is a valence of X, and A denotes an alicyclic skeleton having an epoxy group, represented by formula (II),
which comprises polymerizing a compound represented by formula (III)

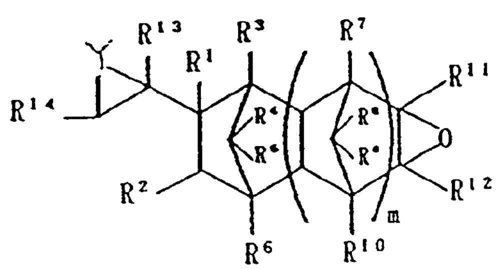

(III)

wherein m is an integer of 0 to 2, Y denotes S or O, and $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ each denote a hydrogen atom or a hydrocarbon group having 1 to 10 carbon atoms.

FIG. 1

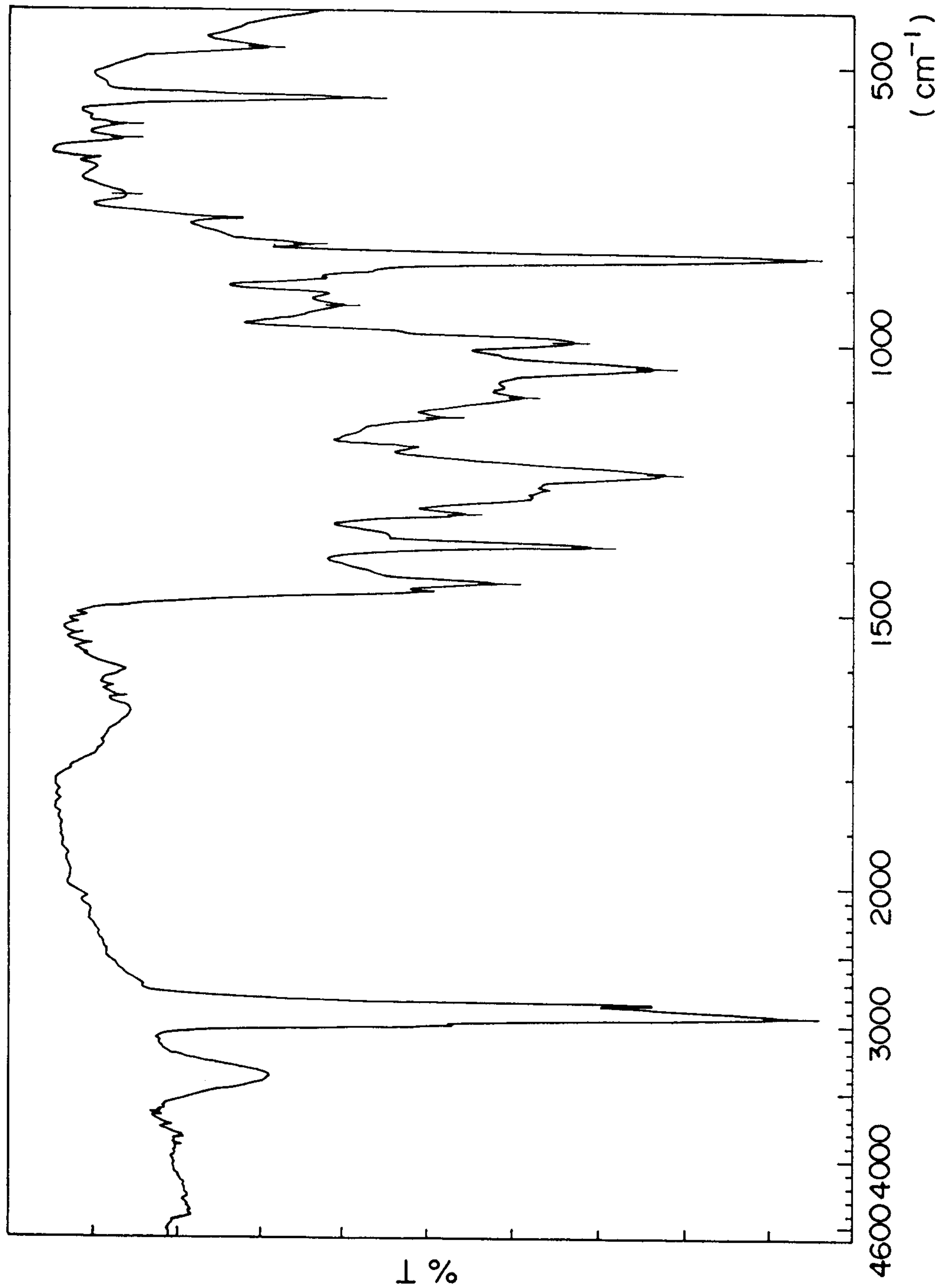

FIG. 2

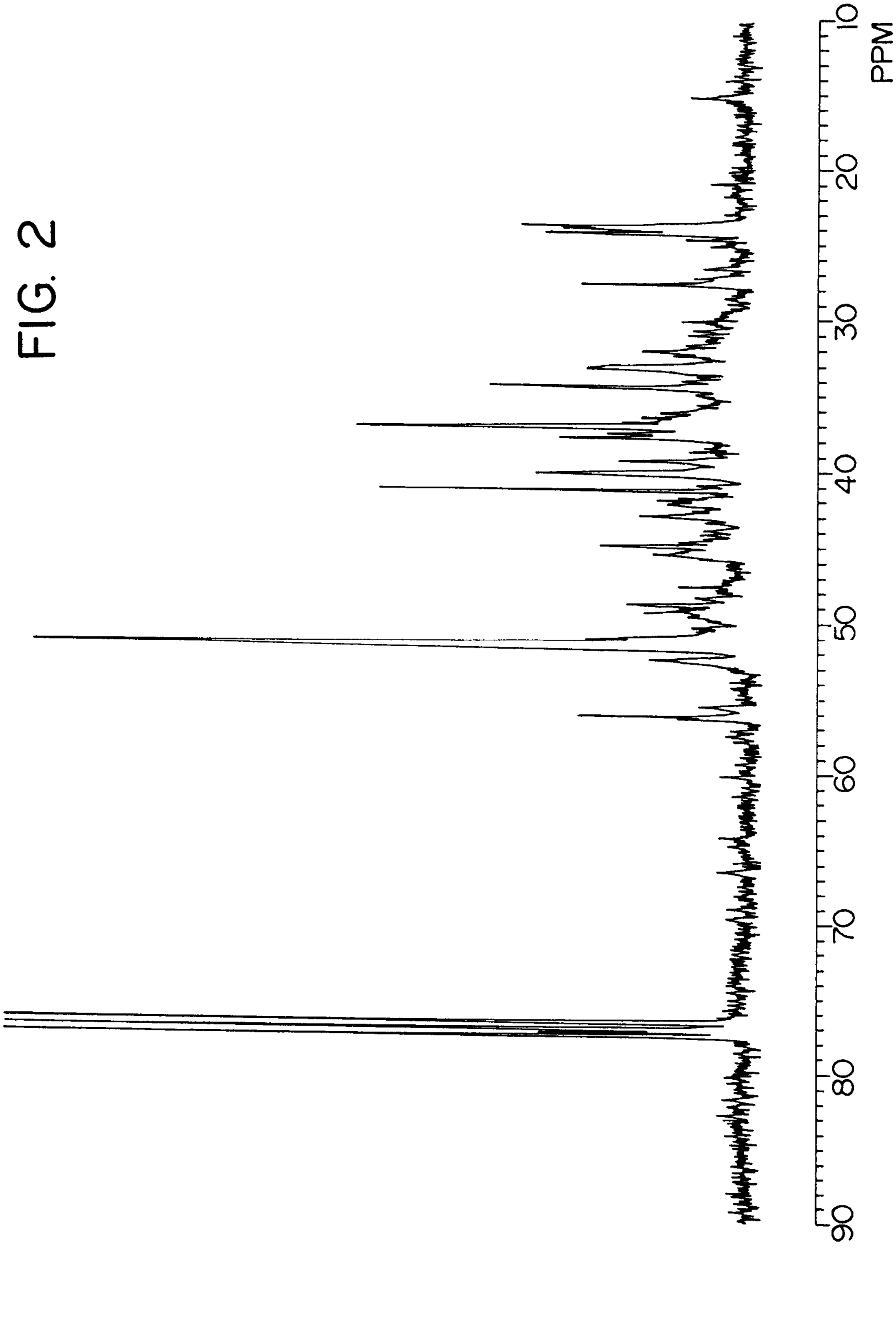

FIG. 3

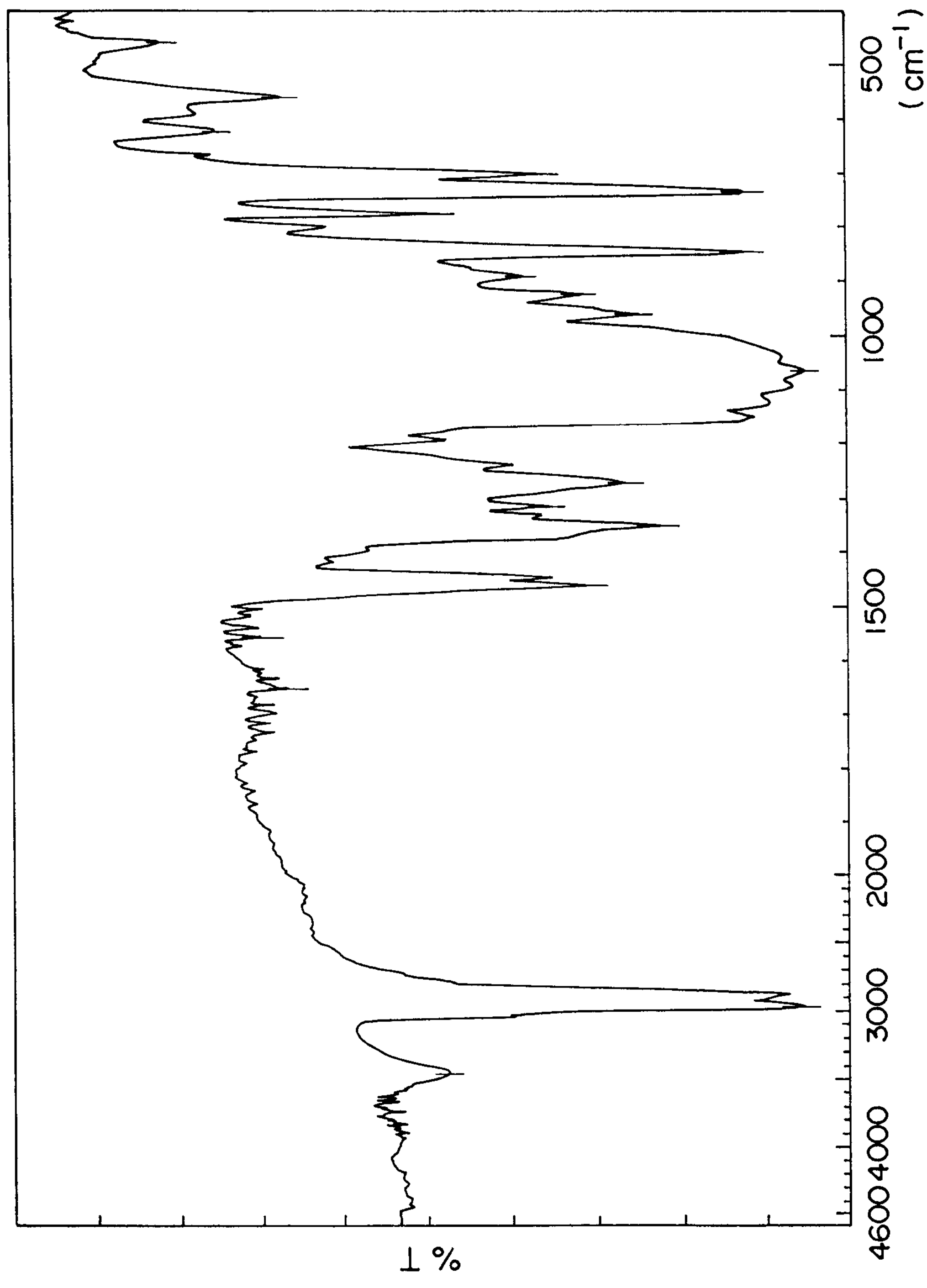

FIG. 4

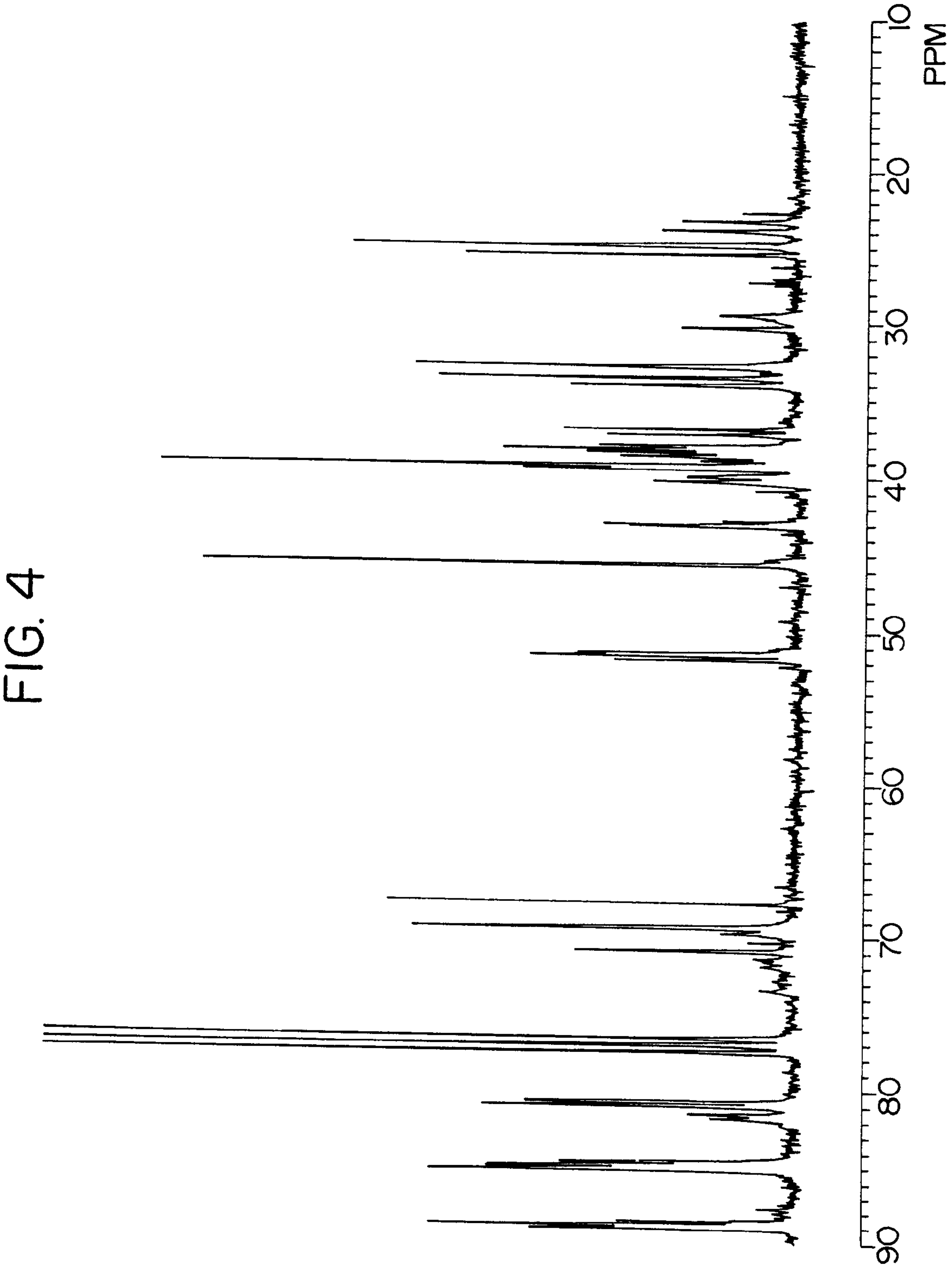

FIG. 5